# EUROPÄISCHE PATENTSCHRIFT

(11) **EP 2 475 990 B1**
(45) Veröffentlichungstag und Bekanntmachung des Hinweises auf die Patenterteilung: **29.04.2015**
(21) Anmeldenummer: 10768392.2
(22) Anmeldetag: 09.09.2010
(51) Int. Cl.: G01N 33/49

(54) **VORRICHTUNG ZUR DURCHFÜHRUNG VON TESTS ZUR HÄMOSTASE**
DEVICE FOR TESTING FOR HEMOSTASIS
DISPOSITIF PERMETTANT DE RÉALISER DES TESTS D'HÉMOSTASE

(30) Priorität: 09.09.2009 DE 102009040880
(43) Veröffentlichungstag der Anmeldung: 18.07.2012
(73) Patentinhaber: Andreas Hettich GmbH & Co. KG, 78532 Tuttlingen (DE)
(72) Erfinder: GEHRING, Frank, K., 72364 Obernheim (DE); MÜLLER, Lothar, 65205 Wiesbaden (DE)
(74) Vertreter: Puschmann Borchert Bardehle Patentanwälte Partnerschaft mbB
(86) Internationale Anmeldenummer: PCT/EP2010/005534
(87) Internationale Veröffentlichungsnummer: WO 2011/029593

(56) Entgegenhaltungen:
- EP-A2- 1 072 887
- JP-A- 2001 108 678
- US-A- 5 892 144
- US-A1- 2002 019 037
- ANDERSSON, GLASMÄSTAR, SUTHERLAND, LIDBERG, KASEMO: "Cell adhesion on supported lipid bilayers", JOURNAL OF BIOMEDICAL MATERIALS RESEARCH A, Bd. 64, Nr. 4, 2003, Seiten 622-629, XP002612669,
- HANSSON, TOSATTI, ISAKSSON, WETTERÖ, TEXTOR, LINDAHL, TENGVALL: "Whole blood coagulation on protein adsorption-resistant PEG and peptide functionalised PEG-coated titanium surfaces", BIOMATERIALS, Bd. 26, 20. Mai 2004 (2004-05-20), Seiten 861-872, XP002612673,

## Beschreibung

Die Erfindung betrifft eine Vorrichtung nach der im Oberbegriff der in Anspruch 1 angegebenen Art.

Eine für die Medizin herausragende Rolle spielt die Analyse von Blut. Anhand dieser sind vielerlei Krankheiten, Anomalien, Infektionen oder Gerinnungsstörungen erkennbar. Diese Analysen werden im Allgemeinen an Proben vorgenommen, die zuerst aus einem Blutkreislauf entnommen und daraufhin in einem Labor weiterverarbeitet werden. Eine besondere Rolle spielt dabei die Analyse der Hämostase. Es sind bereits Vorrichtungen verbreitet, mit welchen ein Patient direkt einen bestimmten Gerinnungswert auslesen kann. Vorrangig trifft dies auf den Quickwert, die Prothrombinzeit zu. Zur Analyse unterschiedlicher Parameter sind bisher separate Vorrichtungen notwendig.

Darüber hinaus sind Vorrichtungen bekannt, die mittels eines Schwingquarzes eine Stoffeigenschaft ermitteln. Diese Technologie ist weitgehend unter "Quarz Chrystal Microbalance" QCM bekannt, wie zum Beispiel die DE 696 101 83 T2. Ein Aspekt der QCM basiert darauf, dass sich an der spezifisch adhäsiven Oberfläche des Quarzes die gewünschten Stoffe anlagern und dadurch die Resonanzfrequenz des Quarzes ändern, wodurch ein Rückschluss auf die Anzahl oder Funktionalität des Stoffes zulässig ist. Weiter sind aus der WO 99 403 97 Quarze bekannt, die mehrere Oszillatoren umfassen und unterschiedliche Beschichtungen zur Detektion unterschiedlicher Stoffe aufweisen.

Die Messung von Blutgerinnung mittels eines Resonators beziehungsweise eines Schwingquarzes ist nach dem Stand der Technik sehr ungenau. Eine Viskosität kann beispielsweise nicht gemessen werden, wenn auf einer Oberfläche eine viskoelastische Schicht entsteht, die akustisch undurchsichtige Eigenschaften aufweist. Dies ist zum Beispiel der Fall, falls diese Schicht zu dick ist, so dass die Eindringtiefe der akustischen Welle nicht mehr das zu vermessende Probenfluid erreicht.

Darüber hinaus besteht auf der anderen Seite das Problem, dass bei einer völlig protein- beziehungsweise zellresistenten Oberfläche die Gerinnung nicht unmittelbar an der Schwingquarzoberfläche stattfindet. Dadurch können die akustischen Wellen nicht tief genug in das zu messende Medium eindringen. In diesem Fall kann die durch die Gerinnung hervorgerufene Viskositätsänderung nicht oder nicht valide gemessen werden. Somit ist es zwar grundsätzlich bekannt, Blutparameter mittels eines Schwingquarzes oder Dickenscherschwingers zu messen, der eine der Blutprobe zugewandten Oberfläche aufweist, wobei die Ergebnisse aber nicht zwingend reproduzierbar sind.

Der Aufsatz "Competitve Protein adsorption on micro patterned polymeric biomaterials, and viscoelastic properties of tailor made extracellular matrices", Biomolecular Engineering 24, (2007) 87-91, Welle et al., befasst sich mit Untersuchung von Oberflächen, insbesondere Polystyrol (PS) Oberflächen und deren Bindungsverhalten gegenüber Proteinen. Gemäß diesem Aufsatz wird festgestellt, dass UV behandeltes (PS) bzw. eine entsprechend gemusterte Oberfläche eine niedrigere Bindungsfähigkeit und geringere Viskosität von oberflächengebundenem Albumin hervorruft. Ein Nachweis dazu wird mit einer QCM-D Technik durchgeführt.

In "Cell adhesion on supported lipid bilayers" werden Biomembranen mit abwechselnden zellresistenten und -adhäsiven Regionen beschrieben. Das an der Oberfläche adsorbierte Material wird mit einer QCU-D Technik gemessen. (J. Biomed. Mat. Res. A, Vol. 64, No. 4, S.622-629).

Die US 2008/0114549 A1 offenbart eine Anordung zur Bestimmung der Gerinnungszeit von Blut. Zur Aktivierung und Beschleunigung der plasmatischen Gerinnung wird Thromboplastin in die Oberfläche eines Schwingquarzes, der zur Messung der Gerinnungszeit eingesetzt wird, integriert. Eine Integration des Aktivators in gemusterter bzw. in streifenförmiger Weise reduziert den Einfluss auf das Schwingungsverhalten des Kristalls.

Der Erfindung liegt die Aufgabe zu Grunde, eine Vorrichtung zur Messung von Parametern der Hämostase anzugeben, mit der sich zuverlässig, genau und schnell unterschiedliche Parameter der primären und sekundären Hämostase bestimmen lassen.

Die Aufgabe wird gemäß dem kennzeichnenden Teil des Anspruchs 1 gelöst.

Erfindungsgemäß umfasst die Vorrichtung zur Messung von Parametern der Hämostase einen Grenzflächensensor, der eine Sensoroberfläche aufweist, die sowohl adhäsive als auch nicht-adhäsive Bereiche gegenüber Blutbestandteilen aufweist.

Die Kombination von adhäsiven und nicht adhäsiven Bereichen der Resonatoroberfläche hat den Vorteil, dass Blutbestandteile eines Probenfluids lediglich an die adhäsiven Bereiche binden und die nicht-adhäsiven Bereiche durch die Ausbildung von Aggregaten und Fibrinnetzen überspannen.

Dadurch erreicht man zuverlässig, dass die Gerinnung unmittelbar an der Sensoroberfläche stattfindet und die durch die Gerinnung hervorgerufene Viskositätsänderung an den akustisch durchsichtigen protein- bzw. zellresistenten Bereichen zu einem aussagekräftigen Sensorsignal führt. Dadurch ist eine zuverlässige und genaue Messung von Hämostase Parametern gewährleistet. Darüber hinaus wird, indem die Blutbestandteile nur an bestimmten Bereichen anbinden können, gewährleistet, dass die an der Sensoroberfläche entstehenden Schichtdicken kleiner als die Eindringtiefe des Grenzflächensensors sind.

Auf die Darstellung der Anregung durch ein Oszillatormodul und der Ausbildung der Schwingungsmessung wird zu Gunsten der Kürze verzichtet. Schwingungsanregung und Messung sind bekannter Stand der Technik und tragen nicht zum Erfindungsgedanken bei, sondern stellen lediglich die Rahmenbedingungen bereit.

In einer weitern vorteilhaften Ausführungsform sind die nicht adhäsiven Bereiche protein- und/oder zellresistent ausgebildet. Dies hat den Vorteil, dass sich Blutbestandteile nicht an diesen Bereichen der Oberfläche anlagern. Adhärieren zu viele Blutbestandteile, kann beispielsweise eine Viskositätsänderung nicht mehr valide gemessen werden, da die Viskosität mit der Adhäsion überlagert ist und/oder die Schicht akustisch undurchsichtig ist.

In einer besonders vorteilhaften Ausführungsform sind die adhäsiven und nicht-adhäsiven Bereiche mosaikförmig auf der Schwingquarzoberfläche angeordnet. Bei einer solchen Oberflächengestaltung können besonders genaue Messungen durchgeführt werden. Eine derartige Bereichsaufteilung gewährleistet eine optimale Verteilung der Ankerstellen, was die Ausbildung einer weitgehend homogenen Schicht fördert.

Insbesondere sind die adhäsiven Bereiche aus Gold und die nicht adhäsiven Bereiche aus Polyethylen (PE) ausgebildet. Eine Ausbildung der Oberfläche aus diesen Materialen, hat den Vorteil, dass sowohl Gold als auch Polyethylen (PE) in der Mikrosystemtechnik weitverbreitete Werkstoffe sind und daher gut bekannt und leicht zu verarbeiten sind. Ein weiterer Vorteil in der Verwendung einer Goldschicht besteht darin, dass diese gleichzeitig als Elektrode des Schwingquarzes dienen kann.

Insbesondere ist die Oberfläche des Schwingquarzes so aufgeteilt, dass die nicht-adhäsiven Bereiche einen Anteil von mindestens 20 Prozent und maximal 90 Prozent der Gesamtfläche des Sensors einnehmen. Innerhalb dieser Grenzen lassen sich die besten Ergebnisse erzielen.

Vorzugsweise ist in die Sensoroberfläche bereits ein Aktivator, wie beispielsweise Thrombin, integriert. Man umgeht damit das Problem, dass der Zeitpunkt von der Aktivierung der Probe bis zur Messung nicht besonders kurz sein muss. Aus diesem Grund kann die gesamte Vorrichtung einfacher gestaltet werden. Der Aktivator kann sowohl auf die adhäsiven Bereiche, als auch die nicht-adhäsiven Bereiche aufgebracht werden.

Die Blutbestandteile adhärieren an der Fibrinogenschicht und werden gleichzeitig aktiviert, wodurch die Aggregation ausgelöst wird. Dadurch kann beispielsweise die Gerinnungszeit vom Zeitpunkt der Blutapplikation bis zur Gerinnung bestimmt werden.

In einer besonders vorteilhaften Ausführungsform ist der Grenzflächensensor als akustischer Resonator ausgebildet. Der Resonator kann alternativ in Form eines Dickenscherschwingers, einer Mikroquarzwaage oder eines Schwingquarzes ausgebildet sein. Die genannten Formen sind in der Analytik weit verbreitet und gut bekannt.

Gemäß einer vorteilhaften Weiterbildung, kann die Resonatoroberfläche auch mehrschichtig ausgebildet sein. Dies empfiehlt sich vor allem bei komplexeren Beschichtungsverfahren.

In einer weiteren Ausführungsform, ist der Grenzflächensensor als optischer Sensor, insbesondere zur Messung der Oberflächen- Plasmon- Resonanz ausgebildet.

Weitere Vorteile, Merkmale und Anwendungsmöglichkeiten der vorliegenden Erfindung ergeben sich aus der nachfolgenden Beschreibung in Verbindung mit dem in der Zeichnung dargestellten Ausführungsbeispiel. Die Erfindung wird im Folgenden anhand der Zeichnung näher beschrieben. In der Beschreibung, in den Patentansprüchen, in der Zusammenfassung und der Zeichnung werden die in der unten aufgeführten Liste der Bezugszeichen verwendeten Begriffe und zugeordneten Bezugszeichen verwendet. In der Zeichnung bedeuten:

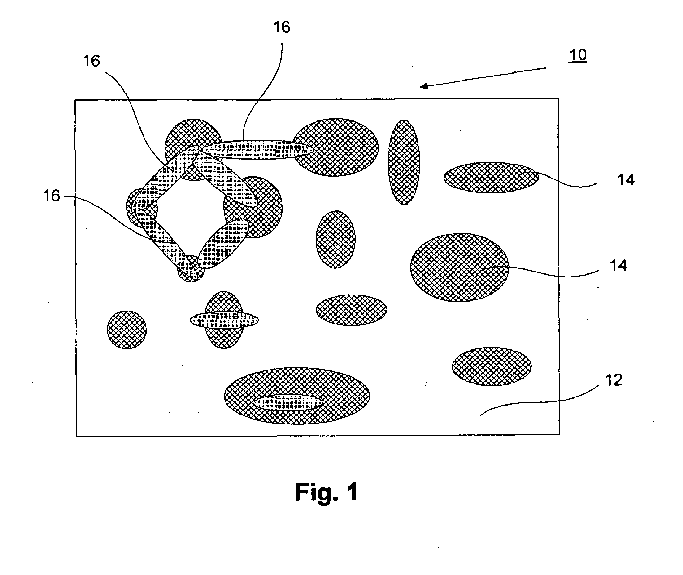

- Fig. 1: eine Schwingquarzoberfläche aus PE und Gold.

Fig. 1 zeigt die Oberfläche eines Schwingquarzes 10, die eine PE-Schicht 12 und eine Goldschicht 14 aufweist. Die PE-Schicht ist in bekannter Weise zellresistent. Sie verhindert daher eine Anlagerung von Proteinen und anderen Zellbestandteilen und Blutbestandteile 16. Die Goldschicht 14 hingegen ist adhäsiv und erlaubt daher eine Anlagerung von Blutbestandteilen in diesem Bereich. Die Blutbestandteile überspannen die nicht-adhäsiven PE-Bereiche. Durch die geringen Anlagerungsmöglichkeiten wird eine Schichtdicke aus Blutbestandteilen gewährleistet, die sowohl eine ausreichende akustische Eindringtiefe gewährleistet, und darüber hinaus durch die gebildeten Ankerstellen der Gerinnungsprozess nahe der Oberfläche des Schwingquarzes ausgelöst wird.

Somit kann neben Parametern der plasmatischen Gerinnung auch die Thrombozytenfunktion bestimmt werden. Dies erlaubt eine Unterscheidung, welcher Gerinnungszweig geschädigt ist.

### Bezugszeichenliste

- 10: Schwingquarz
- 12: PE-Schicht
- 14: Gold-Schicht
- 15: Blutbestandteile

## Patentansprüche

1. Vorrichtung zur Messung von Gerinnungsparametern eines Probenfluids, umfassend einen Grenzflächensensor (10) mit einer Sensoroberfläche, die dem Probenfluid zugewandt ist, wobei der Grenzflächensensor (10) ein Sensorsignal ausgibt, das sich aufgrund der Viskositätsänderung des Probenfluids ergibt, wobei die Sensoroberfläche adhäsive Bereiche (14) und nicht-adhäsive Bereiche (12) gegenüber Blutbestandteilen aufweist,
**dadurch gekennzeichnet, dass** die Vorrichtung eingerichtet ist, aus dem Sensorsignal Gerinnungsparameter des Probenfluids zu bestimmen.

2. Vorrichtung nach Anspruch 1, **dadurch gekennzeichnet, dass** die nicht adhäsiven Bereiche (12) möglichst protein- bzw. zellresistent ausgebildet sind.

3. Vorrichtung nach einem der vorangegangenen Ansprüche, **dadurch gekennzeichnet, dass** die nicht-adhäsiven Bereiche (12) und die adhäsiven Bereiche (14) mosaikförmig auf der Sensoroberfläche angeordnet sind.

4. Vorrichtung nach einem der vorangegangenen Ansprüche, **dadurch gekennzeichnet, dass** die nicht-adhäsive Bereiche aus Polymerbeschichtungen insbesondere Polyethylen (PE) oder Polyethylenglykol (PEG) ausgebildet sind.

5. Vorrichtung nach einem der vorangehenden Ansprüche, **dadurch gekennzeichnet, dass** die adhäsiven Bereiche (14) aus Gold oder Polystyrol (PS), ausgebildet sind.

6. Vorrichtung nach einem der vorangehenden Ansprüche, **dadurch gekennzeichnet, dass** die nicht-adhäsiven Bereiche einen Anteil von mindestens 20% und maximal 90% an der Gesamtfläche einnehmen.

7. Vorrichtung nach einem der vorangehenden Ansprüche, **dadurch gekennzeichnet, dass** ein Aktivator in die Sensorberfläche integriert ist.

8. Vorrichtung nach Anspruch 7, **dadurch gekennzeichnet, dass** die Sensoroberfläche Fibrinogen aufweist.

9. Vorrichtung nach einem der vorangegangenen Ansprüche, **dadurch gekennzeichnet, dass** die Oberfläche mehrschichtig ausgebildet ist.

10. Vorrichtung nach einem der vorangegangenen Ansprüche, **dadurch gekennzeichnet, dass** der Grenzflächensensor als Resonator ausgebildet ist.

11. Vorrichtung nach Anspruch 10, **dadurch gekennzeichnet, dass** der Resonator (10) als akustischer Resonator, insbesondere Schwingquarz, Dickenscherschwinger ausgebildet ist.

12. Vorrichtung nach einem der Ansprüche 1 bis 9, **dadurch gekennzeichnet, dass** der Grenzflächensensor als optischer Sensor insbesondere zur Messung der Oberflächen Plasmon-Resonanz ausgebildet ist.

## Claims

1. Device for measuring the coagulation parameters of a sample fluid, comprising a boundary layer sensor (10) having a sensor surface which faces said sample fluid, wherein said boundary layer sensor (10) outputs a sensor signal which is obtained as a result of the change in viscosity of said sample fluid, said sensor surface including adhesive regions (14) as well as non-adhesive regions (12) with respect to blood components **characterized in that** said device is adapted to determine coagulation parameters of said sample fluid from said sensor signal.

2. The device of claim 1 **characterized in that** said non-adhesive regions (12) are formed so as to be as resistant as possible to protein and/or cells.

3. The device of one of the preceding claims **characterized in that** said non-adhesive regions (12) and said adhesive regions (14) are arranged on said sensor surface in the form of a mosaic.

4. The device of one of the preceding claims **characterized in that** said non-adhesive regions are formed of polymer coatings, in particular polyethylene (PE) or polyethylene glycol (PEG).

5. The device of one of the preceding claims **characterized in that** said adhesive regions (14) are formed of gold or polystyrene (PS).

6. The device of one of the preceding claims **characterized in that** said non-adhesive regions take up at least 20% and a maximum of 90% of the total surface.

7. The device of one of the preceding claims **characterized in that** an activator has been integrated into the sensor surface.

8. The device of claim 7 **characterized in that** said sensor surface includes fibrinogen.

9. The device of one of the preceding claims **characterized in that** said surface is of the multilayer type.

10. The device of one of the preceding claims **characterized in that** said boundary layer sensor is provided in the form of a resonator.

11. The device of claim 10 **characterized in that** said resonator (10) is provided in the form of an acoustic resonator, in particular a quartz oscillator, a thickness shear resonator.

12. The device of one of claims 1 to 9 **characterized in that** said boundary layer sensor is provided in the form of an optical sensor, in particular for measuring the surface plasmon resonance.

## Revendications

1. Dispositif de mesure de paramètres de coagulation d'un échantillon liquide comprenant une sonde d'interface (10) avec une surface de détection dirigée vers l'échantillon liquide, la sonde d'interface (10) émettant un signal lorsque survient une variation en viscosité de l'échantillon liquide, la surface de détection présentant des zones adhésives (14) et des zones non adhésives (12) pour les composés sanguins,
**caractérisé en ce que**
le dispositif est conçu pour le calcul de paramètres de coagulation de l'échantillon liquide sur la base du signal de la sonde.

2. Dispositif selon la revendication 1, **caractérisé en ce que** les zones non adhésives (12) sont agencées pour bénéficier d'une résistance optimale aux protéines et cellules.

3. Dispositif selon l'une des revendications précédentes, **caractérisé en ce que** les zones non adhésives (12) et les zones adhésives (14) sont disposées sur la surface de détection en mosaïque.

4. Dispositif selon l'une des revendications précédentes, **caractérisé en ce que** les zones non adhésives sont composés de revêtements de polymère, notamment de polyéthylène (PE) ou de polyéthylène glycol (PEG).

5. Dispositif selon l'une des revendications précédentes, **caractérisé en ce que** les zones adhésives (14) sont composées d'or ou de polystyrène (PS).

6. Dispositif selon l'une des revendications précédentes, **caractérisé en ce que** les zones non adhésives recouvrent entre 20 et 90 % de la surface totale.

7. Dispositif selon l'une des revendications précédentes, **caractérisé en ce qu'**un activateur est intégré à la surface de détection.

8. Dispositif selon la revendication 7, **caractérisé en ce que** la surface de détection présente des fibrinogènes.

9. Dispositif selon l'une des revendications précédentes, **caractérisé en ce que** la surface est composée de plusieurs couches.

10. Dispositif selon l'une des revendications précédentes, **caractérisé en ce que** la sonde d'interface est agencée comme un résonateur.

11. Dispositif selon la revendication 10, **caractérisé en ce que** le résonateur (10) est agencé comme un résonateur acoustique, plus précisément comme un quartz oscillant, un oscillateur de cisaillement d'épaisseur.

12. Dispositif selon l'une des revendications 1 à 9, **caractérisé en ce que** la sonde d'interface est agencée comme une sonde optique, plus précisément en vue de mesurer la résonance plasmonique de surface.
